# EUROPÄISCHE PATENTSCHRIFT

(11) **EP 0 583 300 B1**
(45) Veröffentlichungstag und Bekanntmachung des Hinweises auf die Patenterteilung: **21.12.1994**
(21) Anmeldenummer: 92909442.3
(22) Anmeldetag: 29.04.1992
(51) Int. Cl.: A61K 7/13

(54) **HAARFÄRBEMITTEL**
HAIR COLOURING AGENT
AGENT DE TEINTURE POUR LES CHEVEUX

(30) Priorität: 08.05.1991 DE 19914115148
(43) Veröffentlichungstag der Anmeldung: 23.02.1994
(73) Patentinhaber: Henkel Kommanditgesellschaft auf Aktien, D-40191 Düsseldorf (DE)
(72) Erfinder: RÖTGERS, Christiane, D-7500 Karlsruhe (DE); HÖFFKES, Horst, D-4000 Düsseldorf 13 (DE); ROSE, David, D-4010 Hilden (DE)

## Beschreibung

Die Erfindung betrifft Oxidationsfärbemittel, enthaltend Oxidationsfarbstoffvorprodukte in einem Träger, die als Oxidationsfarbstoffvorprodukte ein 2,4,5,6-Tetraaminopyrimidin oder ein 6-Hydroxy-2,4,5-Triaminopyridin als Oxidationsbase (Entwickler) sowie eine Kombination aus bestimmten Grünkupplern und Violettkupplern zur Erzeugung besonders brillanter und waschechter Schwarzfärbungen enthalten.

Für das Färben von Keratinfasern, insbesondere von Haaren, spielen die sogenannten Oxidationsfarben, die durch oxidative Kupplung einer oder mehrerer Entwicklerkomponenten (Oxidationsbasen) untereinander oder mit einer oder mehreren Kupplerkomponenten entstehen, weger ihrer intensiven Farben und guten Substantivität für Keratinfasern eine bevorzugte Rolle.

Als Oxidationsbasen werden üblicherweise Paraphenylen-diamin-Derivate, Paraaminophenole, Diaminopyridinderivate, 4-Aminopyrazolonderivate, heterocyclische Hydrazonderivate, 2,4,5,6-Tetraaminopyrimidin oder 6-Hydroxy-2,4,5-Triaminopyrimidin eingesetzt.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen ohne die Kopfhaut zu stark anzufärben. Die damit erzeugten Färbungen müssen eine hohe Stabilität gegen Wärme, Licht und die bei der Dauerwellung des Haares verwendeten Chemikalien aufweisen. Schließlich sollen die Oxidationshaarfarbstoffvorprodukte in toxikologischer und dermatologischer Hinsicht unbedenklich sein.

Besonders vielseitige Oxidationsbasen sind das 2,4,5,6-Tetraaminopyrimidin und das 6-Hydroxy-2,4,5-Triaminopyrimidin. Zur Erzeugung schwarzer Haarfärbungen ist es üblich, diese Oxidationsbasen mit Kupplern zu kombinieren, die bei der oxidativen Kupplung gelbe, rote und blaue Farbstoffe bilden.

Die jeweils verwendeten Blaukuppler, Gelbkuppler und Rotkuppler müssen dabei vergleichbar gutes Aufziehvermögen und die gebildeten Farbstoffe vergleichbare Echtheitseigenschaften aufweisen, damit unter dem Einfluß von Wärme, Licht und Haarwaschmitteln kein selektiver Abbau einer Farbkomponente erfolgt und das Haar seine Farbnuance verändert.

Die derzeit bekannten Kombinationen von Blaukupplern, Gelbkupplern und Rotkupplern für 2,4,5,6-Tetraaminopyrimidin und 6-Hydroxy-2,4,5-Triaminopyrimidin liefern schwarze Haarfärbungen, die nach mehrmaligem Haarewaschen oder unter dem Einfluß von Wärme und Licht eine Farbverschiebung in den Braun- und Rotbereich zeigen.

Es wurde nunmehr gefunden, daß besonders brillante schwarze Färbungen mit deutlich verbesserten Echtheitseigenschaften erzielt werden, wenn man eine Kombination aus wenigstens einer grün kuppelnden und einer violett kuppelnden Kupplerverbindung spezieller Struktur mit einer Oxidationsbase vom Typ des 2,4,5,6-Tetraaminopyrimidins oder des 6-Hydroxy-2,4,5-triaminopyrimidins einsetzt.

Gegenstand der Erfindung sind Oxidationsfärbemittel, enthaltend Oxidationsfarbstoffvorprodukte in eins kosmetischen Träger, die als Oxidationsfarbstoffvorprodukte eine Oxidationsbase der Formel I 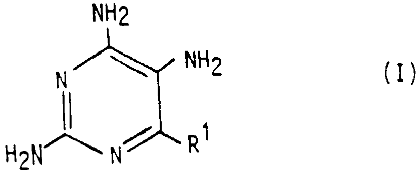 in der R¹ ein NH₂-Gruppe oder eine Hydroxylgruppe ist und eine Kombination aus wenigstens einer Kupplerverbindung der Formel II 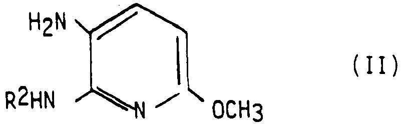 in der R² eine Alkylgruppe mit 1-4 C-Atomen oder eine 2-Methoxyethylgruppe ist und wenigstens einer zweiten Kupplerverbindung der Formel III 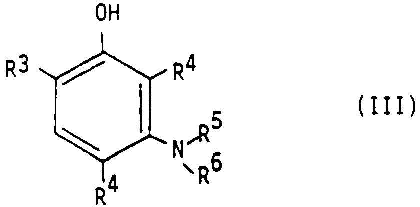 in der R³ = Wasserstoff oder eine CH₃O-Gruppe und R⁴, R⁵ und R⁶ = Wasserstoff oder Alkylgruppen mit 1-4 C-Atomen sind oder R⁵ auch eine Cycloalkylgruppe mit 5 oder 6 C-Atomen ist, enthalten.

Die Oxidationsbase der Formel I, worin R¹ eine Aminogruppe ist, das 2,4,5,6-Tetraaminopyrimidin, und dessen Salze sind aus DE-PS 23 59 399 als Entwicklerkomponenten für 0xidationsfärbemittel bekannt. Die Oxidationsbase der Formel I, worin R¹ eine Hydroxylgruppe ist, das 6-Hydroxy-2,4,5-triaminopyrimidin, ist aus Berichten der Deutschen Chem. Gesellschaft 33 (1900), 1371 -1383 bekannt. Ihre Verwendung als Entwicklerkomponente ist in DE-PS-25 16 118 beschrieben.

Kupplerverbindungen der Formel II und deren Verwendung zur Herstellung von Oxidationsfärbemitteln sind z. B. aus EP 106 987 bekannt. Kupplerverbindungen der Formel III sind m-Aminophenol und die zum Beispiel aus DE-PS 24 47 017 als Violettkuppler für 2,4,5,6-Tetraaminopyrimidin bekannten m-Aminophenolderivate oder die aus DE 38 22 365 A1 bekannten N-Cycloalkyl-3-aminophenole.

Besonders bevorzugt sind erfindungsgemäße Oxidationsfärbemittel, die als Oxidationsbase das 2,4,5,6-Tetraaminopyrimidin oder dessen Salze, als Kuppler der Formel II das 3-Amino-6-methoxy-2-methylaminopyridin und als Kuppler der Formel III das m-Aminophenol enthalten.

Die erfindungsgemäßen Oxidationsfärbemittel erzeugen auf Keratinfasern, insbesondere auf menschlichem Haar, intensive schwarze Färbungen, die sich durch eine hohe Farbechtheit gegenüber Haarvaschmitteln sowie gegenüber Wärme und Sonneneinstrahlung auszeichnen.

In den erfindungsgemhßen Haarfärbemitteln werden die Oxidationsbasen der Formel I und die Kuppler der Formeln II und III bevorzugt im molaren Verhältnis von I:II:III = 2:1:1 eingesetzt, d. h. auf ca. 2 Mol der Oxidationsbasen werden je ein Mol des Grünkupplers der Formel II und 1 Mol des Violettkupplers der Formel III angewendet. Eine Abweichung von diesem Molverhältnis im Bereich von ± 0,3 Mol der Kupplerverbindung pro Mol der Oxidationsbase kann im allgemeinen ohne merkliche Farbverschiebung toleriert werden. Die 0xidationsbase der Formel I sollte in den erfindungsgemäßen Haarfärbemitteln in einer Menge von 5 - 20 mmol, bezogen auf 100 g des Färbemittels, enthalten sein.

Die Verbindungen der Formel I, II und III bilden mit Säuren Salze, sie können sowohl in freier Form als auch in Form ihrer wasserlöslichen Salze eingesetzt werden, z. B. als Hydrochloride, Sulphate, Phosphate, Acetate, Propionate, Lactate oder Citrate. Es ist auch nicht erforderlich, daß die Verbindungen der Formel I, II und III einheitlich zusammengesetzt sind. Vielmehr sind auch Gemische von Verbindungen, die unter die jeweilige allgemeine Formel fallen, zur Ausführung der Erfindung geeignet.

Zur Herstellung der erfindungsgemäßen Haarfärbemittel werden die Oxidationsfarbstoffvorprodukte in einen geeigneten kosmetischen Träger eingearbeitet. Solche Träger sind z. B. Cremes, Emulsionen, Gele oder auch tensidhaltige, schaumende Lösungen, z. B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische, ampholytische oder zwitterionische Tenside, z. B. Seifen, Fettalkoholsulfate, Alkansulfonate, α-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z. B. Methyl- oder Hydroxyethylencellulose, Stärke, Fettkomponenten wie z. B. Fettalkohole, Paraffinöle oder Fettsäureester, ferner Parfümöle und haarpflegende Zusätze, wie z. B. wasserlösliche kationische Polymere, Proteinderivate, Pantothensäure und Cholesterin.

Besonders geeignet als Träger ist ein Gel mit einem Gehalt von 1 - 20 Gew.-% einer Seife, bevorzugt Ammoniumoleat oder eine Öl-in-Wasser-Emulsion mit einem Gehalt von 1 - 25 Gew.-% einer Fettkomponente und 0,5 - 30 Gew.-% eines Emulgiermittels aus der Gruppe der anionischen, nichtionischen, ampholytischen oder zwitterionischen Tenside.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen inbesondere Wasserstoffperoxid oder dessen Gemische aus derartigen Wasserstoffperoxidanlagerungsprodukten mit Kaliumperoxiddisulfat in Betracht.

Bevorzugt wird eine Zubereitung des Oxidationsmittels unmittelbar vor dem Haarefärben mit der Zubereitung aus Oxidationsfarbstoffvorprodukten und Träger vermischt. Das dabei entstehende gebrauchsfertige Haarfärbepräparat sollte bevorzugt einen pH-Wert in Bereichen von 6 - 10 aufweisen.

Besonders bevorzugt ist die Anwendung der Haarfärbemittel in einem schwach alkalischen Milieu. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbshampoo verwendet wurde.

Die nachfolgenden Beispiele sollen den Erfindungsgegenstand näher erläutern ohne ihn jedoch hierauf zu beschränken.

### Beispiele

Es wurden erfindungsgemäße Haarfärbemittel in Form einer Haarfärbe-Cremeemulsion der folgenden Zusammensetzung hergestellt:

### Beispiel 1

| | |
|---|---|
| Fettalkohol C₁₂-C₁₈ | 10 g |
| Fettalkohol C₁₂-C₁₄ + 2 EO-sulfat, Na-Salz (28%ig) | 25 g |
| Wasser | 15 g |
| 2,4,5,6-Tetraaminopyrimidin (E) | 14 mMol |
| 3-Amino-6-methoxy-2-methylamino-pyridin (K1) | 7 mMol |
| m-Aminophenol (K2) | 7 mMol |
| Ascorbinsäure | 0,9 g |
| Ammoniumcarbonat | 0,5 g |
| Histidin | 0,5 g |
| Parfümöl | 0,2 g |
| Ammoniak (25 Gew.-% in H₂O) bis pH = | 9,8 |
| Wasser | ad 100 g |

Die Bestandteile wurden der Reihe nach miteinander vermittelt. Nach Zugabe der Oxidationsfarbstoffvorprodukte und der Hilfsmittel wurde zunächst der pH-Wert mit konzentrierter Ammoniaklösung auf pH = 9,8 eingestellt, dann wurde der Ansatz mit Wasser auf 100 g aufgefüllt.

Die oxidative Entwicklung der Färbung wurde mit 6%iger Wasserstoffperoxidlösung als Oxidationsmittel durchgeführt. Hierzu wurden 100 g der Emulsion mit 50 g Wasserstoffperoxidlösung versetzt und vermischt.

Die Färbecreme wurde auf ca. 5 cm lange Strähnen standardisierten, zu 90 % ergrauten, aber nicht besonders vorbehandelten Menschenhaares aufgetragen und dort 30 Minuten bei 27°C belassen. Nach Beendigung des Färbeprozesses wurde das Haar gespült, mit einem üblichen Haarwaschmittel ausgewaschen und anschließend getrocknet: Es resultierte ein brillant tiefschwarz gefärbtes Haar.

Zur Prüfung der Echtheit der Färbung wurde das gefärbte Haar mehrmals hintereinander mit einem üblichen Haarwaschmittel shampooniert. Nach sechsmaliger Shampoobehandlung betrug die Farbintensität noch 93 % der Ausgangs-Intensität. Es war keine Farbverschiebung in den Rotbereich erkennbar.

In gleicher Weise wurden in die Haarfärbe-Cremeemulsion die folgenden Kupplerpaarungen eingesetzt:

### Beispiel 2

K1 : 3-Amino-6-methoxy-2-methylaminopyridin
K2 : 3-N,N,-Dimethylaminophenol

### Beispiel 3

K1 : 3-Amino-6-methoxy-2-(2-methoxyethylamino)-pyridin
K2 : 5-Amino-2-methoxyphenol

### Beispiel 4

K1 : 3-Amino-6-methoxy-2-methylaminopyridin
K2 : 3-Amino-2,4-dimethylphenol

### Beispiel 5

K1 : 3-Amino-6-methoxy-2-methylaminopyridin
K2 : 3-(N-Cyclopentyl)-aminophenol

In allen Fällen wurden brillant tiefschwarze Haaranfärbungen hoher Waschechtheit erhalten.

## Patentansprüche

1. Oxidationsfärbemittel, enthaltend Oxidationsfarbstoffvorprodukte in einem kosmetischen Träger, **dadurch gekennzeichnet, daß** als Oxidationsfarbstoffvorprodukte eine Oxidationsbase der Formel I 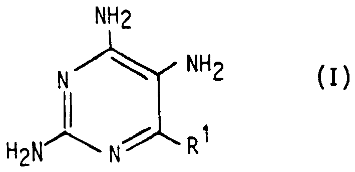 in der R¹ eine -NH₂-Gruppe oder eine Hydroxylgruppe ist und eine Kombination aus wenigstens einer Kupplerverbindung der Formel II 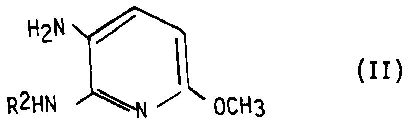 in der R² eine Alkylgruppe mit 1 - 4 C-Atomen oder eine 2-Methoxyethylgruppe ist, und wenigstens einer zweiten Kupplerverbindung der Formel III 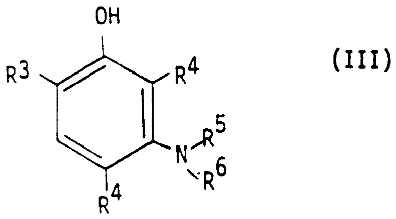 in der R³ = Wasserstoff oder eine Methoxygruppe und R⁴, R⁵ und R⁶ Wasserstoff oder Alkylgruppen mit 1 - 4 C-Atomen sind oder R⁵ auch eine Cycloalkylgruppe mit 5 oder 6 C-Atomen ist.

2. Oxidationsfärbemittel nach Anspruch 1, **dadurch gekennzeichnet, daß** als Oxidationsbase 2,4,5,6-Tetraaminopyrimidin, als Kuppler der Formel (II) 3-Amino-6-methoxy-2-methylaminopyridin und als Kuppler der Formel (III) m-Aminophenol enthalten ist.

3. Oxidationsfärbemittel nach Anspruch 1 oder 2, **dadurch gekennzeichnet, daß** die Oxidationsbase der Formel I und die Kuppler der Formel III und III im molaren Verhältnis I:II:III = 2:1:1 und die Oxidationsbase in einer Menge von 5 - 20 mMol bezogen auf 100 g des Färbemittels enthalten ist.

## Claims

1. Oxidation dyes containing oxidation dye precursors in a cosmetic carrier, **characterized in that** it contains an oxidation base corresponding to formula I: 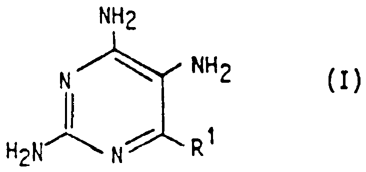 in which R¹ is an -NH₂- group or a hydroxyl group,
and a combination of at least one coupling component corresponding to formula II: 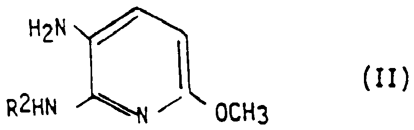 in which R² is an alkyl group containing 1 to 4 C atoms or a 2-methoxyethyl group, and at least one second coupling component corresponding to formula III: 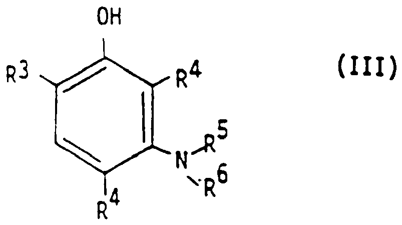 in which R³ is hydrogen or a methoxy group and R⁴, R⁵ and R⁶ are hydrogen or alkyl groups containing 1 to 4 carbon atoms or R⁵ may even be a cycloalkyl group containing 5 or 6 C atoms.

2. An oxidation dye as claimed in claim 1, **characterized in that** 2,4,5,6-tetraaminopyrimidine is used as the oxidation base, 3-amino-6-methoxy-2-methylaminopyridine is used as the coupling component corresponding to formula II and m-aminophenol is used as the coupling component corresponding to formula III.

3. An oxidation dye as claimed in claim 1 or 2, **characterized in that** the oxidation base corresponding to formula I and the coupling components corresponding to formulae II and II are present in a molar ratio of I:I:III = 2:1:1 and the oxidation base is present in a quantity of 5 to 20 mmoles per 100 g of the dye.

## Revendications

1. Agents de teinture par oxydation renfermant des produits précurseurs de colorants par oxydation dans un support cosmétique, **caractérisés en ce que** l'on met en oeuvre comme produits précurseurs de colorant par oxydation, une base d'oxydation de formule (I) : 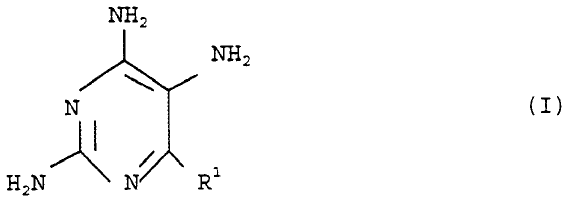 dans laquelle R¹ est un groupe NH₂ ou un hydroxyle, et une combinaison à base d'au moins un composé d'agent de couplage de formule (II) : 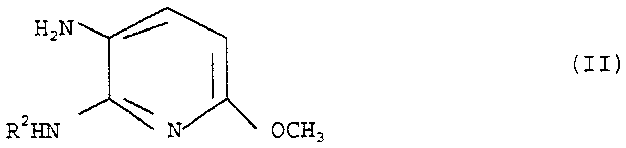 dans laquelle R² est un radical alcoyle ayant de 1 à 4 atomes de carbone ou un groupe méthoxyéthyle et d'au moins un deuxième composé d'agent de couplage de formule (III) : 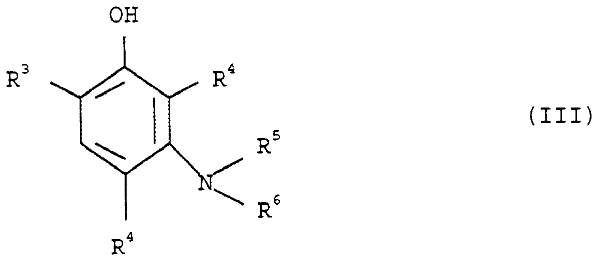 dans laquelle R³ est de l'hydrogène ou un groupe méthoxy, et R⁴, R⁵ et R⁶ sont de l'hydrogène ou un radical alcoyle ayant de 1 à 4 atomes de carbone ou R⁵ est également un radical cycloalcoyle ayant de 5 ou 6 atomes de carbone.

2. Agents de teinture par oxydation selon la revendication 1, **caractérisés en ce que** comme base d'oxydation on inclut la 2,4,5, 6-tétraaminopyrimidine, comme agent de couplage de formule (II) la 3-amino-6-méthoxy-2-méthylaminopyridine et comme agent de couplage de formule (III) le m-aminophénol.

3. Agents de teinture pour oxydation selon la revendication 1 ou 2, **caractérisés en ce que** la base d'oxydation de formule (I) et les agents de couplage de formule (II) et (III) sont contenus dans un rapport molaire (I), (II), (III) = 2 : 1 : 1 et que la base d'oxydation est contenue en quantités allant de 5 à 20 mol rapporté à 100 g d'agent de teinture.
